# EUROPEAN PATENT SPECIFICATION

(11) **EP 3 442 550 B1**
(45) Date of publication and mention of the grant of the patent: **13.03.2024**
(21) Application number: 17716507.3
(22) Date of filing: 06.04.2017
(51) Int. Cl.: A61K 35/745, A23L 33/135, A61P 7/00

(54) **BIFIDOBACTERIA FOR REDUCING FOOD, ENERGY AND/OR FAT INTAKE**
BIFIDOBAKTERIEN ZUR REDUKTION DER LEBENSMITTEL, ENERGIE- UND/ODER FETTAUFNAHME
BIFIDOBACTÉRIES POUR RÉDUIRE LA PRISE D'UN ALIMENT, D'UNE ÉNERGIE ET/OU DE POIDS

(30) Priority: 14.04.2016 EP 16165379
(43) Date of publication of application: 20.02.2019
(73) Proprietor: International N&H Denmark ApS, 2800 Kongens Lyngby (DK)
(72) Inventor: STENMAN, Lotta, 48210 Kotka (FI); LAHTINEN, Sampo, 08350 Lohja (FI)
(74) Representative: International N&H EMEA
(86) International application number: PCT/EP2017/058206
(87) International publication number: WO 2017/178316

(56) References cited:
- WO-A1-2007/043933
- WO-A1-2015/007941
- WO-A2-2010/146568
- L.K. STENMAN ET AL: "Potential probiotic Bifidobacterium animalis ssp. lactis 420 prevents weight gain and glucose intolerance in diet-induced obese mice", BENEFICIAL MICROBES, vol. 5, no. 4, 1 December 2014 (2014-12-01), pages 437-445, XP055296829, NL ISSN: 1876-2883, DOI: 10.3920/BM2014.0014
- ALVIN IBARRA ET AL: "Effects of polydextrose on different levels of energy intake. A systematic review and meta-analysis", APPETITE, vol. 87, 1 April 2015 (2015-04-01), pages 30-37, XP055303986, US ISSN: 0195-6663, DOI: 10.1016/j.appet.2014.12.099

## Description

### FIELD OF THE INVENTION

This invention relates to new uses of a bacterium of the genus *Bifidobacterium,* particularly, but not exclusively, a bacterium of the *Bifidobacterium animalis* subspecies *lactis* strain 420 (B420). This invention also relates to food products, dietary supplements and pharmaceutically acceptable formulations containing said bacterium.

### BACKGROUND OF THE INVENTION

Regulation of energy balance is critical for the survival of an organism. When nutrients are freely available, they are stored to account for low energy intake during times of scarcity. In a normal state the brain, together with energy storage tissues, regulates energy balance by reducing energy intake when energy stores are congested. If the energy storage machinery is disturbed, the brain is no longer able to maintain energy balance. This could lead to inability to maintain adequate energy intake, often manifested as wilting in elderly populations, or to excess storage of energy in adipose tissue and even obesity.

Body mass index (BMI) is a simple index of weight-for-height that is commonly used to classify body weight status in adults. It is defined as a person's weight in kilograms divided by the square of his height in meters (kg/m2).

The World Health Organisation (WHO) definition is a BMI below 18.5 is underweight; a BMI between 18.5 and 24.99 is normal weight; a BMI greater than or equal to 25 is overweight; a BMI greater than or equal to 30 is obesity.

Overweight and obesity are defined as abnormal or excessive fat accumulation. Underweight is defined as a body weight that is too low to maintain normal bodily functions. Both underweight and overweight may lead to disturbances in metabolic functions, such as hormonal signalling.

Once considered a high-income country problem, overweight and obesity are now on the rise in low-and middle-income countries, particularly in urban settings. In developing countries with emerging economies (classified by the World Bank as lower- and middle-income countries) the rate of increase of childhood overweight and obesity has been more than 30% higher than that of developed countries.

Overweight and obesity are linked to more deaths worldwide than underweight. Most of the world's population live in countries where overweight and obesity kill more people than underweight (this includes all high-income and most middle-income countries). The fundamental cause of obesity and overweight is an energy imbalance between calories consumed and calories expended.

The most common consequences of overweight and obesity are diseases such as: cardiovascular diseases (mainly heart disease and stroke), which were the leading cause of death in 2012; diabetes; musculoskeletal disorders (especially osteoarthritis - a highly disabling degenerative disease of the joints); some cancers (endometrial, breast, and colon).

The risk for these diseases increases with an increase in BMI.

Childhood obesity is associated with a higher chance of obesity, premature death and disability in adulthood. But in addition to increased future risks, obese children experience breathing difficulties, increased risk of fractures, hypertension, early markers of cardiovascular disease, insulin resistance and psychological effects.

Overweight and obesity, as well as their related diseases, are largely preventable, and the food industry can play a significant role in the fight against obesity.

It is well known that dysfunctional energy regulation may lead to a variety of metabolic disorders, including obesity. The connections between gut microbiota, energy homeostasis, and the pathogenesis of metabolic disorders are now well-established (Daisuke et al., Front Endocrinol (Lausanne). 2014; 5: 81).

The recent publication *"*Neuronal regulation of Energy Homeostasis: Beyond the Hypothalamus and feeding", Michael J Waterson et al., Cell Metabolism 22, December 1, 2015, stresses the importance of the brain in maintaining energy homeostasis and its relation with obesity and other metabolic diseases.

### SUMMARY OF THE INVENTION

In one aspect, the present invention provides a non-therapeutic use of a bacterium of the genus *Bifidobacterium* or a mixture thereof for reducing food, energy and/or fat intake in a mammal.

In particular, the present invention provides a non-therapeutic use of a bacterium of the genus *Bifidobacterium* or a mixture thereof as probiotics for reducing food, energy and/or fat intake in a mammal.

In particular, the non-therapeutic use of the bacterium of the genus *Bifidobacterium* concerns the *Bifidobacterium* of the *Bifidobacterium animalis* ssp. *lactis* strain 420 (B420).

### Advantages

It has surprisingly been found by the present inventors that treatment with a bacterium of the genus *Bifidobacterium* or a mixture thereof, especially the *Bifidobacterium* of the *Bifidobacterium animalis* ssp. *lactis* strain 420 (B420), shows a reduction in food, energy and/or fat intake in a mammal. This confers the potential for bacterium of the genus *Bifidobacterium* or a mixture thereof, especially the *Bifidobacterium* of the *Bifidobacterium animalis* ssp. *lactis* strain 420 (B420) to be useful in treating and/or preventing a number of obesity-related diseases, such as high blood pressure, diabetes, heart diseases, high cholesterol levels, cancer, infertility, among others, through a physiological mechanism that reduces the energy intake in a mammal.

Without wishing to be bound by theory, it is believed that the *Bifidobacterium* of the present invention, when used alone or in combination with one with one or more fibres and/or prebiotics, has an effect on the gut microbiota, on the brain and on the energy homeostasis, helping control obesity and other metabolic diseases.

### DETAILED DISCLOSURE OF THE INVENTION

### Bacteria

The bacterium used in the present invention is selected from a bacterium of the genus *Bifidobacterium* or a mixture thereof. Preferably the *Bifidobacterium* to be used in the present invention is a *Bifidobacterium* which is generally recognised as safe and, which is preferably GRAS approved. Generally recognized as safe (GRAS) is an American Food and Drug Administration (FDA) designation that a chemical or substance added to food is considered safe by experts, and so is exempted from the usual Federal Food, Drug, and Cosmetic Act (FFDCA) food additive tolerance requirements.

In one embodiment, the present invention relates to a bacterium of the genus *Bifidobacterium* or a mixture thereof for use in reducing food, energy and/or fat intake in a mammal.

In another embodiment, the present invention relates to use of a bacterium of the genus *Bifidobacterium* or a mixture thereof for reducing food, energy and/or fat intake in a mammal

In yet a further embodiment, the present invention relates to use of a bacterium of the genus *Bifidobacterium* or a mixture thereof for the manufacture of a food product, a dietary supplement or a pharmaceutically acceptable formulation for reducing food, energy and/or fat intake in a mammal.

The bacterium may be used in any form capable of exerting the effects described herein. For example, the bacteria may be viable, dormant, inactivated or dead bacteria. Preferably, the bacteria are viable bacteria.

The bacteria may comprise whole bacteria or may comprise bacterial components. Examples of such components include bacterial cell wall components such as peptidoglycan, bacterial nucleic acids such as DNA and RNA, bacterial membrane components, and bacterial structural components such as proteins, carbohydrates, lipids and combinations of these such as lipoproteins, glycolipids and glycoproteins.

The bacteria may also or alternatively comprise bacterial metabolites. In the present specification the term "bacterial metabolites" includes all molecules produced or modified by the (probiotic) bacteria as a result of bacterial metabolism during growth, survival, persistence, transit or existence of bacteria during the manufacture of the probiotic product and storage and during gastrointestinal transit in a mammal. Examples include all organic acids, inorganic acids, bases, proteins and peptides, enzymes and co-enzymes, amino acids and nucleic acids, carbohydrates, lipids, glycoproteins, lipoproteins, glycolipids, vitamins, all bioactive compounds, metabolites containing an inorganic component, and all small molecules, for example nitrous molecules or molecules containing a sulphurous acid.

Preferably the bacteria comprise whole bacteria, more preferably whole viable bacteria.

Preferably, the *Bifidobacterium* used in accordance with the present invention is one which is suitable for human and/or animal consumption. A skilled person will be readily aware of specific species and or strains of *Bifidobacteria* from within the genera described herein which are used in the food and/or agricultural industries and which are generally considered suitable for human and/or animal consumption.

In the present invention, the *Bifidobacterium* used may be of the same type (species and strain) or may comprise a mixture of species and/or strains.

Suitable bacteria are selected from the species *Bifidobacterium lactis, Bifidobacterium bifidium, Bifidobacterium longum, Bifidobacterium animalis, Bifidobacterium breve, Bifidobacterium infantis, Bifidobacterium catenulatum, Bifidobacterium pseudocatenulatum, Bifidobacterium adolescentis, and Bifidobacterium angulatum,* and combinations of any thereof.

Preferably, the *Bifidobacterium* used in the present invention is of the species *Bifidobacterium animalis.* More preferably, the *Bifidobacferium* used in the present invention is of the *Bifidobacterium animalis* ssp. *lactis.*

In a particularly preferred embodiment, the bacteria used in the present invention are *Bifidobacterium animalis* ssp. *lactis* strain 420 (B420). This strain is commercially available from DuPont Nutrition Biosciences ApS.

This strain of *Bifidobacterium animalis* ssp. *lactis* has also been deposited under the reference DGCC420 by DuPont Nutrition Biosciences ApS, of Langebrogade 1, DK-1411 Copenhagen K, Denmark, in accordance with the Budapest Treaty on 30 June 2015 at the Leibniz-Institut Deutsche Sammlung von Mikroorganismen und Zellkulturen GmbH (DSMZ), Inhoffenstrasse 7B, 38124 Braunschweig, Germany, where it is recorded under registration number DSM 32073. It is requested that the biological material shall be made available only by the issue of a sample to an expert nominated by the requester.

In one embodiment, the bacterium used in the present invention is a probiotic bacterium. In this specification the term 'probiotic bacterium' is defined as covering any non-pathogenic bacterium which, when administered live in adequate amounts, confer a health benefit on the host. These probiotic strains generally have the ability to survive the passage through the upper part of the digestive tract. They are non-pathogenic, non-toxic and exercise their beneficial effect on health on the one hand via ecological interactions with the resident flora in the digestive tract, and on the other hand via their ability to influence the immune system in a positive manner via the "GALT" (gut-associated lymphoid tissue). Depending on the definition of probiotics, these bacteria, when given in a sufficient number, have the ability to progress live through the intestine, however they do not cross the intestinal barrier and their primary effects are therefore induced in the lumen and/or the wall of the gastrointestinal tract. They then form part of the resident flora during the administration period. This colonization (or transient colonization) allows the probiotic bacteria to exercise a beneficial effect, such as the repression of potentially pathogenic micro-organisms present in the flora and interactions with the immune system of the i ntesti ne.

In preferred embodiments, the bacterium used in the present invention is a probiotic *Bifidobacterium.*

### Dosage

The *Bifidobacterium,* such as a strain of *Bifidobacterium animalis* ssp. *lactis,* for example *Bifidobacterium animalis* ssp. *lactis* strain 420 (B420), used in accordance with the present invention may comprise from 10⁶ to 10¹² CFU of bacteria/g of support, and more particularly from 10⁸ to 10¹² CFU of bacteria/g of support, preferably 10⁹ to 10¹² CFU/g for the lyophilized form.

Suitably, the *Bifidobacterium,* such as a strain of *Bifidobacterium animalis* ssp. *lactis,* for example *Bifidobacterium animalis* ssp. *lactis* strain 420 (B420), may be administered at a dosage of from about 10⁶ to about 10¹² CFU of microorganism/dose, preferably about 10⁸ to about 10¹² CFU of microorganism/dose. By the term "per dose" it is meant that this amount of microorganism is provided to a subject either per day or per intake, preferably per day. For example, if the microorganism is to be administered in a food product, for example in a yoghurt, then the yoghurt will preferably contain from about 10⁸ to 10¹² CFU of the microorganism. Alternatively, however, this amount of microorganism may be split into multiple administrations each consisting of a smaller amount of microbial loading - so long as the overall amount of microorganism received by the subject in any specific time, for instance each 24-hour period, is from about 10⁶ to about 10¹² CFU of microorganism, preferably 10⁸ to about 10¹² CFU of microorganism.

In accordance with the present invention an effective amount of at least one strain of a microorganism may be at least 10⁶ CFU of microorganism/dose, preferably from about 10⁶ to about 10¹² CFU of microorganism/dose, preferably about 10⁸ to about 10¹² CFU of microorganism/dose.

In one embodiment, preferably the *Bifidobacterium,* such as a strain of *Bifidobacterium animalis* ssp. *lactis,* for example *Bifidobacterium animalis* ssp. *lactis* strain 420 (B420), may be administered at a dosage of from about 10⁶ to about 10¹² CFU of microorganism/day, preferably about 10⁸ to about 10¹² CFU of microorganism/day. Hence, the effective amount in this embodiment may be from about 10⁶ to about 10¹² CFU of microorganism/day, preferably about 10⁸ to about 10¹² CFU of microorganism/day.

CFU stands for "colony-forming units". By 'support' is meant the food product, dietary supplement or the pharmaceutically acceptable formulation.

In one embodiment, the present invention relates to a bacterium of the genus *Bifidobacterium* or a mixture thereof, such as a strain of *Bifidobacterium animalis* ssp. *lactis,* for example *Bifidobacterium animalis* ssp. *lactis* strain 420 (B420), in the form of a food product, a dietary supplement or a pharmaceutically acceptable formulation.

### Effects/Subiects/Medical indications

The *Bifidobacteria* to which the present invention relates are administered to a mammal, including for example livestock (including cattle, horses, pigs and sheep), and humans. In some aspects of the present invention the mammal is a companion animal (including pets), such as a dog or a cat for instance. In some aspects of the present invention, the subject may suitably be a human.

The *Bifidobacteria* to which the present invention relates may be suitable for reducing food, energy intake in mammals.

Although birds and poultry, including chickens, are technically not mammals, the present invention may also be suitable for birds and any type of poultry, such as chickens.

The *Bifidobacteria* to which the present invention relates is also suitable for reducing fat intake in mammals.

The *Bifidobacteria* to which the present invention relates may also be suitable for reducing simultaneously food, energy and fat intake in mammals.

In this specification the term "reducing food intake and/or fat intake" refers to any administration of the *Bifidobacteria* according to the present invention and includes reduction of the number of calories absorbed by the mammal, birds or poultry.

In particular, the *Bifidobacteria* according to the present invention is suitable for mammals, birds and poultry ingesting a high-fat diet. This aspect is discussed in more detail below.

### Diet

As noted above, subject mammals, birds or poultry treated with bacteria according to the present invention may ingest a high-fat diet while mitigating the metabolic consequences of their condition(s). In this specification the term 'high-fat diet' means a diet generally containing at least 20%, preferably at least 25%, such as at least 30%, for example at least 35%, such as at least 40%, for example at least 45%, such as at least 50%, for example at least 55%, such as at least 60%, for example at least 65%, such as at least 70%, for example at least 75%, such as at least 80%, for example at least 85%, such as at least 90% of calories from fat.

In some embodiments, mammals, birds or poultry treated with bacteria according to the present invention may ingest a low-carbohydrate diet during the course of the treatment. In this specification the term 'low-carbohydrate diet' means a diet generally containing no greater than 50%, such as no greater than 45%, for example no greater than 40%, such as no greater than 35%, for example no greater than 30%, such as no greater than 25%, for example no greater than 20%, such as no greater than 15%, for example no greater than 10%, such as no greater than 5%, for example no greater than 2%, such as no greater than 1 %, for example no greater than 0.5%, such as no greater than 0.2% of calories from carbohydrate.

### Compositions

While is it possible to administer *Bifidobacferia* alone according to the present invention (i.e. without any support, diluent or excipient), the *Bifidobacteria* are typically and preferably administered on or in a support as part of a product, in particular as a component of a food product, a dietary supplement or a pharmaceutical formulation. These products typically contain additional components well known to those skilled in the art.

Any product which can benefit from the composition may be used in the present invention. These include but are not limited to foods, particularly fruit conserves and dairy foods and dairy food-derived products, and pharmaceutical products. The *Bifidobacteria* may be referred to herein as "the composition of the present invention" or "the composition".

### Food

In one embodiment, the *Bifidobacteria* are employed according to the invention in a food product, such as a food supplement, a drink or a powder based on milk. Here, the term "food" is used in a broad sense and covers food for humans as well as food for animals (i.e. a feed). In a preferred aspect, the food is for human consumption.

The food may be in the form of a solution or as a solid, depending on the use and/or the mode of application and/or the mode of administration.

When used as, or in the preparation of, a food, such as functional food, the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the composition of the present invention can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified coffee beverage.

The composition can further be used as an ingredient in food products such as American cheese sauce, anti-caking agent for grated & shredded cheese, chip dip, cream cheese, dry blended whip topping fat free sour cream, freeze/thaw dairy whipping cream, freeze/thaw stable whipped topping, low fat and light natural cheddar cheese, low fat Swiss style yoghurt, aerated frozen desserts, hard pack ice cream, label friendly, improved economics & indulgence of hard pack ice cream, low fat ice cream: soft serve, barbecue sauce, cheese dip sauce, cottage cheese dressing, dry mix Alfredo sauce, mix cheese sauce, dry mix tomato sauce and others.

The term "dairy product" as used herein is meant to include a medium comprising milk of animal and/or vegetable origin. As milk of animal origin there can be mentioned cow's, sheep's, goat's or buffalo's milk. As milk of vegetable origin there can be mentioned any fermentable substance of vegetable origin which can be used according to the invention, in particular originating from soybeans, rice or cereals.

Still more preferably the food product employed according to the invention is a fermented milk or humanized milk.

For certain aspects, preferably the present invention may be used in connection with yoghurt production, such as fermented yoghurt drink, yoghurt, drinking yoghurt, cheese, fermented cream, milk based desserts and others.

Suitably, the composition can be further used as an ingredient in one or more of cheese applications, meat applications, or applications comprising protective cultures.

The present invention also provides a method of preparing a food or a food ingredient, the method comprising admixing the composition according to the present invention with another food ingredient.

Advantageously, the present invention relates to products that have been contacted with the composition of the present invention, and optionally with other components/ingredients, wherein the composition is used in an amount to be capable of improving the nutrition and/or health benefits of the product.

As used herein the term "contacted" refers to the indirect or direct application of the composition of the present invention to the product. Examples of the application methods which may be used, include, but are not limited to, treating the product in a material comprising the composition, direct application by mixing the composition with the product, spraying the composition onto the product surface or dipping the product into a preparation of the composition.

Where the product of the invention is a foodstuff, the composition of the present invention is preferably admixed with the product. Alternatively, the composition may be included in the emulsion or raw ingredients of a foodstuff. In a further alternative, the composition may be applied as a seasoning, glaze, colorant mixture, and the like.

For some applications, it is important that the composition is made available on or to the surface of a product to be affected/treated. This allows the composition to impart one or more of the following favourable characteristics: nutrition and/or health benefits.

The compositions of the present invention may be applied to intersperse, coat and/or impregnate a product with a controlled amount of a microorganism.

Preferably, the composition is used to ferment milk or sucrose fortified milk or lactic media with sucrose and/or maltose where the resulting media containing all components of the composition - i.e. said microorganism according to the present invention - can be added as an ingredient to yoghurt milk in suitable concentrations - such as for example in concentrations in the final product which offer a daily dose of 10⁶ - 10¹⁰ CFU. The microorganism according to the present invention may be used before or after fermentation of the yoghurt.

For some aspects the microorganisms according to the present invention are used as, or in the preparation of, animal feeds, such as livestock feeds, in particular poultry (such as chicken) feed, or pet food.

Advantageously, where the product is a food product, the *Bifidobacteria* should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Food ingredient

The composition of the present invention may be used as a food ingredient and/or feed ingredient.

As used herein the term "food ingredient" or "feed ingredient" includes a formulation which is or can be added to functional foods or foodstuffs as a nutritional supplement.

The food ingredient may be in the form of a solution or as a solid, depending on the use and/or the mode of application and/or the mode of administration.

### Food Supplements

The composition of the present invention may be - or may be added to - dietary supplements, also referred to herein as food supplements.

Here, the term "dietary supplement" is a product intended for ingestion that contains a "dietary ingredient" intended to add further nutritional value to (supplement) the diet. A "dietary ingredient" may be one, or any combination, of the following substances: a vitamin, a mineral, an herb or other botanical, an amino acid, a dietary substance for use by people to supplement the diet by increasing the total dietary intake, a concentrate, metabolite, constituent, or extract.

Dietary supplements may be found in many forms such as tablets, capsules, soft gels, gel caps, liquids, or powders. Some dietary supplements can help ensure that you get an adequate dietary intake of essential nutrients; others may help you reduce your risk of disease.

### Functional Foods

The composition of the present invention may be - or may be added to - functional foods.

As used herein, the term "functional food" means food which is capable of providing not only a nutritional effect, but is also capable of delivering a further beneficial effect to consumer.

Accordingly, functional foods are ordinary foods that have components or ingredients (such as those described herein) incorporated into them that impart to the food a specific functional - e.g. medical or physiological benefit - other than a purely nutritional effect.

Although there is no legal definition of a functional food, most of the parties with an interest in this area agree that they are foods marketed as having specific health effects beyond basic nutritional effects.

Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

### Pharmaceutical

The composition of the present invention may be used as - or in the preparation of - a pharmaceutical formulation. Here, the term "pharmaceutical" is used in a broad sense - and covers pharmaceuticals for humans as well as pharmaceuticals for animals (i.e. veterinary applications). In a preferred aspect, the pharmaceutical is for human use and/or for animal husbandry.

A pharmaceutically acceptable formulation or support may be for example a formulation or support in the form of compressed tablets, tablets, capsules, ointments, suppositories or drinkable solutions. Other suitable forms are provided below.

When used as - or in the preparation of - a pharmaceutical, the composition of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a solution or as a solid - depending on the use and/or the mode of application and/or the mode of administration.

The *Bifidobacteria* of the present invention may be used as pharmaceutical ingredients. Here, the composition may be the sole active component or it may be at least one of a number (i.e. 2 or more) of active components.

The pharmaceutical ingredient may be in the form of a solution or as a solid - depending on the use and/or the mode of application and/or the mode of administration.

The *Bifidobacteria* may be used according to the present invention in any suitable form - whether when alone or when present in a combination with other components or ingredients. The *Bifidobacteria* used in the present invention may be referred to herein as "the composition". Likewise, combinations comprising the composition of the present invention and other components and/or ingredients (i.e. ingredients - such as food ingredients, functional food ingredients or pharmaceutical ingredients) may be used in any suitable form.

The *Bifidobacteria* may be used according to the present invention in the form of solid or liquid preparations or alternatives thereof. Examples of solid preparations include, but are not limited to tablets, capsules, dusts, granules and powders which may be wettable, spray-dried or freeze-dried. Examples of liquid preparations include, but are not limited to, aqueous, organic or aqueous-organic solutions, suspensions and emulsions.

Suitable examples of forms include one or more of: tablets, pills, capsules, ovules, solutions or suspensions, which may contain flavouring or colouring agents, for immediate-, delayed-, modified-, sustained-, pulsed- or controlled-release applications.

By way of example, if the composition of the present invention is used in a tablet form - such for use as a functional ingredient - the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethrai fatty acid esters, hydroxymethylceilulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

The forms may also include gelatin capsules; fibre capsules, fibre tablets etc.; or even fibre beverages.

Further examples of form include creams. For some aspects the microorganism used in the present invention may be used in pharmaceutical and/or cosmetic creams such as sun creams and/or after-sun creams for example.

In one aspect, the composition according to the present invention may be administered in an aerosol, for example by way of a nasal spray, for instance for administration to the respiratory tract.

### Prebiotics

In one embodiment, the bacterium of the present invention may contain one or more fibres and/or prebiotics.

Prebiotics are a category of functional food, defined as non-digestible food ingredients that beneficially affect the host by selectively stimulating the growth and/or activity of one or a limited number of bacteria (particularly, although not exclusively, probiotics, *Bifidobacteria* and/or lactic acid bacteria) in the colon, and thus improve host health. Typically, prebiotics are carbohydrates (such as oligosaccharides), but the definition does not preclude non-carbohydrates. The most prevalent forms of prebiotics are nutritionally classed as soluble fibres. To some extent, many forms of dietary fibres exhibit some level of prebiotic effect.

In one embodiment, a prebiotic is a selectively fermented ingredient that allows specific changes, both in the composition and/or activity in the gastrointestinal microflora that confers benefits upon host well-being and health.

Suitably, the prebiotic may be used according to the present invention in an amount of 0.01 to 100 g/day, preferably 0.1 to 50 g/day, more preferably 0.5 to 20 g/day. In one embodiment, the prebiotic may be used according to the present invention in an amount of 1 to 10 g/day, preferably 2 to 9 g/day, more preferably 3 to 8 g/day. In another embodiment, the prebiotic may be used according to the present invention in an amount of 5 to 50 g/day, preferably 10 to 25 g/day.

Examples of dietary sources of prebiotics include soybeans, inulin sources (such as Jerusalem artichoke, jicama, and chicory root), raw oats, unrefined wheat, unrefined barley and yacon.

Examples of suitable prebiotics include alginate, xanthan, pectin, locust bean gum (LBG), inulin, guar gum, galacto-oligosaccharide (GOS), fructo-oligosaccharide (FOS), polydextrose (i.e. Litesse^{®}), lactitol, lactosucrose, soybean oligosaccharides, isomaltulose (Palatinose TM), isomalto-oligosaccharides, gluco-oligosaccharides, xylooligosaccharides, manno-oligosaccharides, beta-glucans, cellobiose, raffinose, gentiobiose, melibiose, xylobiose, cyciodextrins, isomaltose, trehalose, stachyose, panose, pullulan, verbascose, galactomannans, and all forms of resistant starches.

A particularly preferred example of a prebiotic is polydextrose.

In some embodiments, a combination of *Bifidobacterium* and one or more fibres and/or prebiotics according to the present invention exhibits a synergistic effect in certain applications (i.e. an effect which is greater than the additive effect of the bacteria when used separately). Without wishing to be bound by theory, it is believed that such a combination is capable of selectively stimulating the growth and/or activity of the *Bifidobacteria* in the colon, and thus improving its effect and the host health.

In one embodiment, the *Bifidobacterium* or a mixture thereof used in the combination with one or more fibres and/or prebiotic is of the species *Bifidobacterium animalis.* More preferably, the *Bifidobacterium* or a mixture thereof used in the combination with one or more fibres and/or prebiotic is of the *Bifidobacterium animalis* ssp. *lactis.*

In a particularly preferred embodiment, the *Bifidobacterium* or a mixture thereof used in the combination with one or more fibres and/or prebiotic is of the *Bifidobacterium animalis* ssp. *lactis* strain 420 (B420).

Suitably, the fibre and/or prebiotic used in the combination is polydextrose.

In another embodiment, the fibre and/or prebiotic used in the combination is Litesse^{®}.

In a further aspect, the invention comprises a food product comprising a *Bifidobacterium* or mixture thereof and one or more fibres and/or a prebiotic.

In a yet further aspect, the invention comprises a food product comprising the *Bifidobacterium* or mixture thereof of the species *Bifidobacterium animalis* and one or more fibres and/or a prebiotic.

In a yet further aspect, the invention comprises a food product comprising the *Bifidobacterium* or mixture thereof of the *Bifidobacterieum animalis* ssp. *lactis* and one or more fibres and/or a prebiotic.

In a yet further aspect, the invention comprises a food product comprising the *Bifidobacterium* or mixture thereof of the *Bifidobacterium animalis* ssp. *lactis* strain 420 (B420) and one or more fibres and/or a prebiotic.

In a further aspect, the invention comprises a dietary supplement comprising a *Bifidobacterium* or mixture thereof and one or more fibres and/or a prebiotic.

In a yet further aspect, the invention comprises a dietary supplement comprising the *Bifidobacterium* or mixture thereof of the species *Bifidobacterium animalis* and one or more fibres and/or a prebiotic.

In a yet further aspect, the invention comprises a dietary supplement comprising the *Bifidobacterium* or mixture thereof of the *Bifidobacterieum animalis* ssp. *lactis* and one or more fibres and/or a prebiotic.

In a yet further aspect, the invention comprises a dietary supplement comprising the *Bifidobacterium* or mixture thereof of the *Bifidobacterium animalis* ssp. *lactis* strain 420 (B420) and one or more fibres and/or a prebiotic.

In a further aspect, the invention comprises a pharmaceutically acceptable formulation comprising a *Bifidobacterium* or mixture thereof and one or more fibres and/or a prebiotic.

In a yet further aspect, the invention comprises a pharmaceutically acceptable formulation comprising the *Bifidobacterium* or mixture thereof of the species *Bifidobacterium animalis* and one or more fibres and/or a prebiotic.

In a yet further aspect, the invention comprises a pharmaceutically acceptable formulation comprising the *Bifidobacterium* or mixture thereof of the *Bifidobacterieum animalis* ssp. *lactis* and one or more fibres and/or a prebiotic.

In a yet further aspect, the invention comprises a pharmaceutically acceptable formulation comprising the *Bifidobacterium* or mixture thereof of the *Bifidobacterium animalis* ssp. *lactis* strain 420 (B420) and one or more fibres and/or a prebiotic.

### Examples

### Materials and methods

### Clinical study design and screening criteria

The intervention was a double-blind, randomized, placebo-controlled, multi-centre parallel study, conducted according to Good Clinical Practice and the Declaration of Helsinki.

A cohort of 225 adults were selected from 263 overweight and obese adults at four research centres in southern Finland and randomized according to a 1:1:1:1 allocation to one of four groups:
1) Placebo (microcrystalline cellulose), 12 g/day;
2) Polydextrose, 12 g/day;
3) Probiotic B420 (*Bifidobacterium* animalis ssp. lactis 420), 10¹⁰ CFU/day; or
4) B420 and polydextrose, 10¹⁰ CFU/day + 12 g/day.

The products for the study were provided in a sachet that the participant mixed with a 250 ml fruit smoothie once per day at the time of their liking for six months.

All randomized participants were 18-65 years old with a Body Mass Index (BMI, calculated as body weight [kg] divided by height [m] squared) between 28.0-34.9 and a waist-hip ratio of ≥0.88 for males and ≥0.83 for females. The most important exclusion criteria included diagnosed metabolic diseases or the use of related medications; use of laxatives, fibre supplements or probiotics in the previous 6 weeks; history of bariatric surgery; use of anti-obesity drugs in the previous 3 months, recent (past 2 months) or on-going use of antimicrobials; on-going or recent participation in a weight-loss program; weight change of 3 kg during previous 3 months; and pregnancy.

### Recruitment and study populations

Before unblinding the study, 209 participants were selected from the 225 participants and were placed into an Intention-to-Treat (ITT) population. The ITT population contained all 209 participants who were assessed for any parameter after the baseline visit.

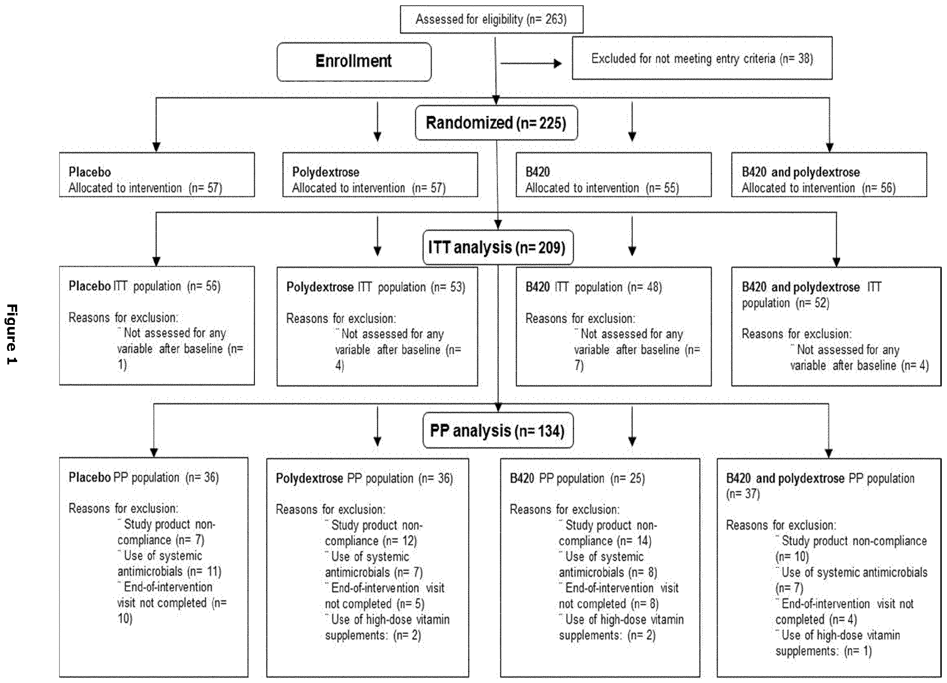

Of the 209 participants in the Intention-to-Treat (ITT) group, 134 participants completed the intervention period with at least 80% study product compliance, and did not use systemic antimicrobials or high-dose vitamin supplements during the intervention (Per protocol population (PP)). Therefore, the PP population better represents the efficacy of the product used in the study (Figure 1).

Figure 1 shows that before unblinding the study, participants were divided into an Intention-to-Treat (ITT) population and a Per Protocol (PP) population according to adherence to the study protocol. Several reasons may apply to a single excluded individual (n represents the number of people involved in each step of the process).

### Dietary intake assessment

The participants filled in a 5-day food diary prior to the baseline, 2-month and end-of-intervention (6-month) clinic visits. Qualified nutritionists analyzed the food diary data with AivoDiet software (Aivo Finland Oy, Finland) using a national database of food ingredients and their compositions (Fineli, National Institute of Health and Welfare, Finland). Because not all study participants recorded the fruit smoothie vehicle in the food diary, data were recorded without the fruit smoothie. The energy content (130 kcal/day) of the fruit smoothie was later added to the energy intake data of all groups and all visits after baseline. The participant data for baseline body weight, height and age were used to calculate basal metabolic rate (BMR), as shown below:
- Women: BMR=655,0955+(9,5634*Body weight(kg))+(1,8496*Body height(cm))-(4,6756*Age in years) kcal/day
- Men: BMR=66,4730+(13,7516*Body weight(kg))+(5,0033*Body height(cm))-(6.755*Age in years) kcal/day

Basal metabolic rate is the amount of energy expended while at rest in a neutrally temperate environment, in the post-absorptive state. Food diaries with energy intake below 80% of the basal metabolic rate for women and 85% for men were regarded as underreported and consequently excluded from the analyses (Figure 1).

### Statistical analysis

The mean change from baseline in all three active groups (groups taking B420, B420 and polydextrose together and polydextrose alone) was compared to placebo as an overall effect (one-way analysis of covariance, using baseline values as covariate). The three active groups were then compared to placebo separately using Dunnett's test, which corrects for multiple comparisons. All analyses were conducted with SAS analysis software, version 9.3. In the ITT population, missing observations were handled with the Last Observation Carried Forward method. In statistics, Dunnett's test is a multiple comparison procedure developed by Canadian statistician Charles Dunnett to compare each of a number of treatments with a single control. Multiple comparisons to a control are also referred to as many-to-one comparisons. The Last Observation Carried Forward method means that for missing values the latest measured value from a previous time point was used in the analysis. A P-value below 0.05 was considered statistically significant, meaning that the hypothesis of the compared observations being different is true with a 95% probability. A P-value above 0.05 does not prove that there was no difference, but rather there is not enough statistical power to draw conclusions with confidence.

### Results

There was a statistically significant overall effect of the study products on energy intake in the PP population (P=0.0054, active groups vs. placebo) (Table 2), but not in the ITT population (P=0.23, active groups vs. placebo) (Table 1). However, differences in the ITT population showed a very similar pattern as in the PP population.

In the PP population, energy intake was statistically significantly reduced by B420 alone (-318.9 kcal/day, P=0.037, B420 vs. placebo) and the combination of B420 with polydextrose (-227 kcal/day, P=0.041), compared with placebo (-23,1 kcal/day) during the 6-month intervention. Polydextrose seemed to decrease energy intake (-200,6 kcal/day), but was not statistically significantly different from placebo (P=0.16, polydextrose vs. placebo) (Table 2).

Absolute fat intake was also statistically significantly different in the active groups compared to the placebo group in the PP population (P=0.008, active groups vs. placebo) (Table 4), but not in the ITT population (P=0.21, active groups vs. placebo), although changes in the ITT population did reflect those seen in the PP population (Table 3). This difference in fat intake was statistically significant in the group taking B420 alone (-21,6 g/day, P=0.03) with a similar trend in the combination of B420 with polydextrose (-10,1 g/day, P=0.11) and polydextrose alone (-11,6 g/day, P=0.17) groups compared to placebo (-2.2 g/day) (Table 4).

There was a borderline non-significant trend towards a decreased dietary proportion of fat in the active groups in the PP population (P=0.058, active groups vs. placebo) (Table 6). A "trend" means a P-value between 0.05-0.10 or sometimes, sometimes up to 0.15. There was no statistically significant overall difference in the ITT population (P=0.47, active groups vs. placebo) (Table 5), results in the ITT population seemed to reflect the changes seen in the PP population.

The decreased proportion of fat in the diet was mostly evident in the PP population in the group taking B420 only (-4.0%, P=0.11) compared to placebo (-0.6%) (Table 6). The other groups showed a smaller and statistically non-significant difference compared to placebo (polydextrose: -2.0%, P=0.39; B420 and polydextrose: -1.7%, P=0.52) Decreasing dietary fat intake is linked to a healthier lifestyle and may help reduce the risk of metabolic disorders.

The fact that differences were much greater in the PP population than in the ITT population indicates that the positive effect was due to the product used in the study. This is because the PP population included only those who were compliant with the protocol and used at least 80% of the study product, whereas the ITT population includes also those who were poorly complying and did not use the study product at all or in adequate amounts.

**Table 1: Energy intake (kcal) in the Intention-to-Treat population**

| | | *Energy (kcal)* | | | | | | | | *Change from baseline (kcal)* | | | | | | | |
|---|---|---|---|---|---|---|---|---|---|---|---|---|---|---|---|---|---|
| | | *n* | *Mean* | *SD* | *Min* | *Q1* | *Median* | *Q3* | *Max* | *n* | *Mean* | *SD* | *Min* | *Q1* | *Median* | *Q3* | *Max* |
| *Product* | *Visit* | | | | | | | | | | | | | | | | |
| *B420* | *Baseline* | 45 | 2169.4 | 442.2 | 1415.6 | 1958.9 | 2084.0 | 2389.0 | 2980.5 | 0 | . | . | . | . | . | . | . |
| | *month 2* | 41 | 2253.2 | 479.3 | 1270.1 | 2042.2 | 2179.1 | 2478.7 | 3750.8 | 40 | 62.0 | 467.2 | -615.0 | -254.2 | -29.3 | 290.6 | 1681.2 |
| | *month 6* | 36 | 2039.5 | 550.9 | 1297.0 | 1601.2 | 2005.3 | 2324.7 | 3737.8 | 35 | **-190.0** | 473.3 | -952.0 | -543.9 | -255.3 | 34.1 | 1051.1 |
| *B420 and polydextrose* | *Baseline* | 50 | 2077.8 | 625.8 | 1142.1 | 1692.0 | 1982.4 | 2141.4 | 4033.5 | 0 | . | . | . | . | . | . | . |
| | *month 2* | 47 | 1980.0 | 438.2 | 1261.6 | 1633.8 | 1959.6 | 2322.7 | 2771.0 | 46 | -113.7 | 523.7 | -1736 | -457.3 | -4.4 | 208.1 | 810.5 |
| | *month 6* | 41 | 1898.9 | 474.0 | 1159.2 | 1613.4 | 1873.4 | 2083.5 | 3599.1 | 39 | **-123.9** | 634.6 | -2160 | -289.0 | -51.0 | 220.6 | 1122.2 |
| *Polydextrose* | *Baseline* | 50 | 2211.4 | 593.4 | 1280.9 | 1753.2 | 2149.7 | 2515.3 | 4545.2 | 0 | . | . | . | . | . | . | . |
| | *month 2* | 50 | 2197.3 | 598.4 | 1345.9 | 1799.1 | 2097.9 | 2470.2 | 4606.1 | 48 | 2.9 | 461.3 | -704.9 | -438.8 | 28.3 | 292.1 | 1435.8 |
| | *month 6* | 49 | 2050.3 | 518.8 | 1259.7 | 1730.3 | 2027.7 | 2299.1 | 3537.6 | 48 | **-160.5** | 466.4 | -1142 | -515.5 | -191.7 | 138.6 | 1093.5 |
| *Placebo* | *Baseline* | 53 | 2144.9 | 571.9 | 1293.7 | 1767.0 | 2010.6 | 2418.0 | 4005.5 | 0 | . | . | . | . | . | . | . |
| | *month 2* | 51 | 2241.8 | 526.6 | 1356.7 | 1954.5 | 2115.6 | 2461.4 | 3948.6 | 48 | 87.8 | 576.2 | -1988 | -171.6 | 129.1 | 449.1 | 1162.0 |
| | *month 6* | 49 | 2115.6 | 448.4 | 1384.3 | 1822.9 | 2065.4 | 2353.9 | 3542.8 | 46 | **-4.4** | 539.2 | -1961 | -189.5 | 13.2 | 180.9 | 1813.0 |

| | | | | | | | | | | | | | | | | | |
|---|---|---|---|---|---|---|---|---|---|---|---|---|---|---|---|---|---|
| No statistically significant differences. Only changes from baseline to month 6 were statistically compared. n: number of observations Mean: the average energy intake or change in energy intake calculated based on the number of participants in the corresponding group and corresponding visit displayed in columns "Product" and "Visit". SD: Standard deviation; Min: Minimum value; Q1: First quartile; Q3: Third quartile; Max: Maximum value. | | | | | | | | | | | | | | | | | |

**Table 2: Energy intake (kcal) in the Per Protocol population**

| | | *Energy (kcal)* | | | | | | | | *Change from baseline (kcal)* | | | | | | | |
|---|---|---|---|---|---|---|---|---|---|---|---|---|---|---|---|---|---|
| | | *n* | *Mean* | *SD* | *Min* | *Q1* | *Median* | Q3 | *Max* | *n* | *Mean* | *SD* | *Min* | *Q1* | *Median* | Q3 | *Max* |
| *Product* | *Visit* | | | | | | | | | | | | | | | | |
| *B420* | *Baseline* | 24 | 2203.7 | 380.6 | 1451.6 | 1997.1 | 2168.7 | 2453.9 | 2980.5 | 0 | . | . | . | . | . | . | . |
| | *month 2* | 22 | 2133.7 | 383.7 | 1270.1 | 1812.0 | 2121.5 | 2478.7 | 2699.5 | 22 | -115.3 | 253.6 | -443.6 | -326.5 | -203.7 | 42.4 | 486.5 |
| | *month 6* | 22 | 1904.7 | 371.4 | 1297.0 | 1587.7 | 1930.0 | 2210.1 | 2528.7 | 22 | **-318.9*** | 303.1 | -787.0 | -543.9 | -319.5 | -147.2 | 519.0 |
| *B420 and polydextrose* | *Baseline* | 35 | 2092.3 | 643.2 | 1142.1 | 1661.9 | 1995.8 | 2141.4 | 4033.5 | 0 | | | | ....... | | | |
| | *month 2* | 34 | 1986.7 | 435.5 | 1261.6 | 1643.6 | 1932.6 | 2297.0 | 2771.0 | 33 | -110.1 | 534.8 | -1736 | -395.3 | -17.3 | 208.1 | 628.4 |
| | *month 6* | 31 | 1866.3 | 489.7 | 1243.6 | 1489.0 | 1870.8 | 2000.4 | 3599.1 | 29 | -227.0* | 640.5 | -2160 | -324.1 | -98.8 | 62.4 | 1122.2 |
| *Polydextrose* | *Baseline* | 33 | 2214.8 | 653.5 | 1280.9 | 1726.6 | 2234.3 | 2526.1 | 4545.2 | 0 | . | . | . | . | . | . | . |
| | *month 2* | 35 | 2165.9 | 637.4 | 1367.2 | 1747.0 | 2092.8 | 2289.4 | 4606.1 | 33 | -15.1 | 425.3 | -704.9 | -455.4 | 26.1 | 249.2 | 1059.7 |
| | *month 6* | 34 | 2004.0 | 521.2 | 1259.7 | 1667.2 | 2018.1 | 2260.6 | 3537.6 | 33 | **-200.6** | 508.8 | -1142 | -682.3 | -191.0 | 105.9 | 1093.5 |
| *Placebo* | *Baseline* | 33 | 2237.7 | 512.1 | 1397.5 | 1868.5 | 2159.1 | 2492.7 | 3838.8 | 0 | | . | . | . | . | . | . |
| | *month 2* | 34 | 2274.8 | 569.2 | 1455.8 | 1946.9 | 2246.3 | 2613.6 | 3948.6 | 31 | 56.8 | 508.0 | -1534 | -182.1 | 60.1 | 364.4 | 1162.0 |
| | *month 6* | 36 | 2182.1 | 463.6 | 1384.3 | 1881.4 | 2096.5 | 2540.5 | 3542.8 | 33 | **-23.1** | 599.9 | -1961 | -189.5 | 2.5 | 148.3 | 1813.0 |

| | | | | | | | | | | | | | | | | | |
|---|---|---|---|---|---|---|---|---|---|---|---|---|---|---|---|---|---|
| * = significant difference from Placebo (Dunnett's test, corrected for multiple comparisons). Only changes from baseline to month 6 were statistically compared. n: number of observations; Mean: the average energy intake or change in energy intake calculated based on the number of participants in the corresponding group and corresponding visit displayed in columns "Product" and "Visit". SD: Standard deviation; Min: Minimum value; Q1: First quartile; Q3: Third quartile; Max: Maximum value. | | | | | | | | | | | | | | | | | |

**Table 3: Fat intake (g) in the Intention-to-Treat population**

| | | *Fat (g)* | | | | | | | | *Change from baseline (g)* | | | | | | | |
|---|---|---|---|---|---|---|---|---|---|---|---|---|---|---|---|---|---|
| | | *n* | *Mean* | *SD* | *Min* | *Q1* | *Median* | Q3 | *Max* | *n* | *Mean* | *SD* | *Min* | *Q1* | *Median* | *Q3* | *Max* |
| *Product* | *Visit* | | | | | | | | | | | | | | | | |
| *B420* | *Baseline* | 45 | 92.8 | 24.1 | 43.4 | 80.7 | 88.7 | 105.8 | 151.1 | 0 | . | . | . | . | . | . | . |
| | *month 2* | 41 | 91.6 | 27.0 | 45.2 | 72.9 | 88.4 | 108.3 | 169.9 | 40 | -2.6 | 24.9 | -48.2 | -18.0 | -5.6 | 15.8 | 59.6 |
| | *month 6* | 36 | 79.1 | 25.5 | 43.3 | 57.6 | 69.0 | 98.6 | 126.2 | 35 | **-16.8** | 23.5 | -62.2 | -32.1 | -18.0 | -7.1 | 38.9 |
| *B420 and polydextrose* | *Baseline* | 50 | 86.4 | 31.7 | 33.8 | 66.6 | 78.5 | 98.2 | 178.7 | 0 | . | . | . | . | . | . | . |
| | *month 2* | 47 | 79.2 | 24.9 | 42.5 | 59.6 | 78.9 | 104.3 | 127.7 | 46 | -7.9 | 28.5 | -90.1 | -20.6 | 1.1 | 11.7 | 41.2 |
| | *month 6* | 41 | 76.9 | 30.1 | 32.9 | 55.8 | 74.4 | 90.6 | 158.6 | 39 | -4.0 | 29.7 | -80.4 | -17.9 | -5.7 | 6.1 | 58.0 |
| *Polydextrose* | *Baseline* | 50 | 91.8 | 27.8 | 46.1 | 73.4 | 90.7 | 104.0 | 195.8 | 0 | . | . | . | . | . | . | . |
| | *month 2* | 50 | 92.4 | 29.4 | 47.9 | 72.6 | 90.5 | 99.4 | 215.3 | 48 | 1.4 | 27.6 | -43.6 | -18.0 | -3.0 | 23.7 | 94.1 |
| | *month 6* | 49 | 81.5 | 25.7 | 42.3 | 62.2 | 79.5 | 91.0 | 168.3 | 48 | **-10.0** | 25.1 | -53.1 | -28.4 | -7.1 | 4.9 | 62.8 |
| *Placebo* | *Baseline* | 53 | 90.0 | 32.7 | 40.4 | 68.6 | 86.9 | 103.4 | 194.6 | 0 | | . | . | . | . | . | . |
| | *month 2* | 51 | 87.8 | 28.6 | 43.7 | 69.0 | 82.2 | 94.7 | 176.4 | 48 | -2.6 | 32.9 | -110.1 | -17.4 | 1.7 | 16.4 | 58.4 |
| | *month 6* | 49 | 86.5 | 29.3 | 41.4 | 70.3 | 81.2 | 98.5 | 210.2 | 46 | **-1.9** | 37.2 | -114.8 | -19.8 | -5.2 | 13.8 | 152.6 |

| | | | | | | | | | | | | | | | | | |
|---|---|---|---|---|---|---|---|---|---|---|---|---|---|---|---|---|---|
| No statistically significant differences. Only changes from baseline to month 6 were statistically compared. n: number of observations; Mean: the average energy intake or change in energy intake calculated based on the number of participants in the corresponding group and corresponding visit displayed in columns "Product" and "Visit". SD: Standard deviation; Min: Minimum value; Q1: First quartile; Q3: Third quartile; Max: Maximum value. | | | | | | | | | | | | | | | | | |

**Table 4: Fat intake (g) in the Per Protocol population**

| | | *Fat (g)* | | | | | | | | *Change from baseline (g)* | | | | | | | |
|---|---|---|---|---|---|---|---|---|---|---|---|---|---|---|---|---|---|
| | | *n* | *Mean* | *SD* | *Min* | *Q1* | *Median* | *Q3* | *Max* | *n* | *Mean* | *SD* | *Min* | *Q1* | *Median* | Q3 | *Max* |
| *Product* | *Visit* | | | | | | | | | | | | | | | | |
| *B420* | *Baseline* | 24 | 93.5 | 21.6 | 45.1 | 81.9 | 91.0 | 105.7 | 146.1 | 0 | . | . | . | . | . | . | . |
| | *month 2* | 22 | 86.9 | 26.3 | 45.2 | 64.8 | 84.0 | 105.8 | 136.6 | 22 | -9.7 | 18.4 | -45.1 | -20.2 | -12.1 | -0.9 | 37.5 |
| | *month 6* | 22 | 73.6 | 21.7 | 43.3 | 57.0 | 67.5 | 88.8 | 116.5 | 22 | -21.6* | 18.8 | -62.2 | -33.7 | -21.1 | -9.5 | 14.9 |
| *B420 and polydextrose* | *Baseline* | 35 | 87.3 | 31.9 | 33.8 | 66.3 | 81.0 | 110.4 | 162.7 | 0 | . | . | . | . | . | . | . |
| | *month 2* | 34 | 78.6 | 25.6 | 42.5 | 59.6 | 72.5 | 99.1 | 127.7 | 33 | -8.6 | 28.7 | -90.1 | -19.7 | 0.9 | 10.2 | 27.5 |
| | *month 6* | 31 | 74.1 | 30.9 | 32.9 | 52.3 | 69.1 | 80.6 | 158.6 | 29 | **-10.1** | 28.5 | -80.4 | -20.6 | -7.4 | 3.3 | 58.0 |
| *Polydextrose* | *Baseline* | 33 | 92.2 | 29.0 | 46.1 | 73.4 | 91.9 | 103.3 | 195.8 | 0 | . | . | . | . | . | . | . |
| | *month 2* | 35 | 92.9 | 30.8 | 47.9 | 71.7 | 89.3 | 99.8 | 215.3 | 33 | 2.2 | 23.3 | -42.6 | -14.3 | -2.7 | 23.3 | 48.3 |
| | *month 6* | 34 | 80.0 | 27.3 | 42.3 | 57.8 | 75.9 | 90.1 | 168.3 | 33 | **-11.6** | 24.4 | -51.8 | -28.0 | -12.2 | 3.2 | 62.8 |
| *Placebo* | *Baseline* | 33 | 96.1 | 32.0 | 44.7 | 76.4 | 94.0 | 110.5 | 194.6 | 0 | . | . | . | . | . | . | . |
| | *month 2* | 34 | 89.9 | 32.3 | 44.5 | 68.8 | 82.5 | 94.7 | 176.4 | 31 | -4.9 | 31.9 | -108.0 | -22.6 | -6.2 | 13.8 | 58.4 |
| | *month 6* | 36 | 91.8 | 31.3 | 41.4 | 73.9 | 83.9 | 107.6 | 210.2 | 33 | -2.2 | 42.8 | -114.8 | -24.9 | -2.6 | 16.8 | 152.6 |

| | | | | | | | | | | | | | | | | | |
|---|---|---|---|---|---|---|---|---|---|---|---|---|---|---|---|---|---|
| * = significant difference from Placebo (Dunnett's test, corrected for multiple comparisons) | | | | | | | | | | | | | | | | | |

Only changes from baseline to month 6 were statistically compared. n: number of observations; Mean: the average energy intake or change in energy intake calculated based on the number of participants in the corresponding group and corresponding visit displayed in columns "Product" and "Visit". SD: Standard deviation; Min: Minimum value; Q1: First quartile; Q3: Third quartile; Max: Maximum value.

**Table 5: Fat intake (%kcal) in the Intention-to-Treat population**

| | | *Fat intake* (% *of kcal)* | | | | | | | | *Change from baseline* (% *of kcal)* | | | | | | | |
|---|---|---|---|---|---|---|---|---|---|---|---|---|---|---|---|---|---|
| | | *n* | *Mean* | *SD* | *Min* | *Q1* | *Median* | *Q3* | *Max* | *n* | *Mean* | *SD* | *Min* | *Q1* | *Median* | Q3 | *Max* |
| *Product* | *Visit* | | | | | | | | | | | | | | | | |
| *B420* | *Baseline* | 45 | 38.3% | 5.0% | 26.0% | 35.0% | 38.4% | 41.5% | 47.2% | 0 | . | . | . | . | . | . | . |
| | *month 2* | 41 | 36.3% | 5.9% | 22.9% | 32.3% | 36.1% | 40.8% | 50.0% | 40 | -2.2% | 6.2% | -14.3% | -6.3% | -2.2% | 1.4% | 11.0% |
| | *month 6* | 36 | 34.8% | 6.1% | 24.5% | 30.3% | 34.1% | 37.9% | 48.7% | 35 | -3.8% | 6.2% | -15.5% | -7.9% | -4.1% | 0.2% | 14.1% |
| *B420 and polydextrose* | *Baseline* | 50 | 37.1% | 6.2% | 25.5% | 32.5% | 36.9% | 41.1% | 55.3% | 0 | . | . | . | . | . | . | . |
| | *month 2* | 47 | 35.7% | 6.2% | 19.4% | 31.2% | 35.3% | 40.5% | 50.1% | 46 | -1.4% | 7.0% | -21.5% | -5.2% | -1.2% | 2.4% | 13.9% |
| | *month 6* | 41 | 35.6% | 6.7% | 19.9% | 31.3% | 35.8% | 38.4% | 53.9% | 39 | -0.3% | 5.7% | -7.9% | -5.5% | -0.9% | 2.1% | 16.3% |
| *Polydextrose* | *Baseline* | 50 | 37.3% | 5.0% | 25.3% | 34.4% | 37.5% | 40.5% | 47.7% | 0 | . | . | . | . | . | . | . |
| | *month 2* | 50 | 37.7% | 4.7% | 29.4% | 34.0% | 38.1% | 41.2% | 49.1% | 48 | 0.4% | 6.4% | -12.9% | -5.1% | -0.6% | 5.4% | 13.2% |
| | *month 6* | 49 | 35.5% | 4.0% | 27.3% | 32.7% | 36.1% | 37.3% | 43.8% | 48 | **-1.7%** | 6.2% | -14.7% | -5.6% | -2.0% | 1.5% | 17.4% |
| *Placebo* | *Baseline* | 53 | 37.2% | 6.3% | 23.3% | 33.9% | 36.8% | 41.7% | 56.7% | 0 | . | . | . | . | . | . | |
| | *month 2* | 51 | 35.0% | 5.9% | 26.9% | 31.6% | 34.2% | 37.2% | 54.9% | 48 | -2.2% | 5.5% | -14.1% | -6.0% | -2.8% | 1.5% | 10.7% |
| | *month 6* | 49 | 36.3% | 6.6% | 26.1% | 31.4% | 35.2% | 40.0% | 57.1% | 46 | -0.8% | 7.5% | -16.5% | -4.7% | -1.5% | 2.4% | 23.4% |

| | | | | | | | | | | | | | | | | | |
|---|---|---|---|---|---|---|---|---|---|---|---|---|---|---|---|---|---|
| No statistically significant differences. Only changes from baseline to month 6 were statistically compared. n: number of observations; Mean: the average energy intake or change in energy intake calculated based on the number of participants in the corresponding group and corresponding visit displayed in columns "Product" and "Visit". SD: Standard deviation; Min: Minimum value; Q1: First quartile; Q3: Third quartile; Max: Maximum value. | | | | | | | | | | | | | | | | | |

**Table 6: Fat intake (%kcal) in the Per Protocol population**

| | | *Fat intake (% of kcal)* | | | | | | | | *Change from baseline* (% *of kcal)* | | | | | | | |
|---|---|---|---|---|---|---|---|---|---|---|---|---|---|---|---|---|---|
| | | *n* | *Mean* | *SD* | *Min* | *Q1* | *Median* | *Q3* | *Max* | *n* | *Mean* | *SD* | *Min* | *Q1* | *Median* | *Q3* | *Max* |
| *Product* | *Visit* | | | | | | | | | | | | | | | | |
| *B420* | *Baseline* | 24 | 38.0% | 4.8% | 27.9% | 34.8% | 37.6% | 41.4% | 47.2% | 0 | . | . | . | . | . | . | . |
| | *month 2* | 22 | 36.3% | 6.6% | 22.9% | 31.7% | 36.9% | 41.5% | 50.0% | 22 | -2.4% | 5.6% | -14.0% | -6.3% | -2.1% | -0.1% | 10.2% |
| | *month 6* | 22 | 34.5% | 6.0% | 24.5% | 30.1% | 33.9% | 38.4% | 46.2% | 22 | -4.0% | 5.9% | -15.5% | -8.1% | -3.2% | 1.4% | 3.9% |
| *B420 and polydextrose* | *Baseline* | 35 | 37.2% | 6.4% | 25.6% | 32.5% | 37.3% | 40.7% | 55.3% | 0 | . | . | . | . | . | . | . |
| | *month 2* | 34 | 35.2% | 6.5% | 19.4% | 30.9% | 33.8% | 39.5% | 50.1% | 33 | -1.7% | 6.5% | -17.6% | -6.2% | -2.1% | 1.1% | 13.9% |
| | *month 6* | 31 | 34.7% | 6.3% | 19.9% | 30.4% | 34.7% | 37.1% | 53.9% | 29 | -1.2% | 5.4% | -7.9% | -5.5% | -2.0% | 1.4% | 16.3% |
| *Polydextrose* | *Baseline* | 33 | 37.5% | 4.7% | 26.6% | 34.5% | 37.4% | 40.9% | 45.5% | 0 | . | . | . | . | . | . | . |
| | *month 2* | 35 | 38.5% | 4.7% | 30.8% | 34.7% | 38.6% | 41.6% | 49.1% | 33 | 0.9% | 6.1% | -12.9% | -5.1% | 0.6% | 5.1% | 12.3% |
| | *month 6* | 34 | 35.4% | 4.0% | 27.3% | 32.7% | 36.1% | 37.3% | 43.8% | 33 | -2.0% | 5.6% | -11.3% | -5.7% | -3.2% | 1.4% | 13.0% |
| *Placebo* | *Baseline* | 33 | 38.2% | 7.0% | 23.3% | 34.6% | 38.4% | 42.0% | 56.7% | 0 | . | . | . | . | . | . | . |
| | *month 2* | 34 | 35.2% | 6.8% | 26.9% | 30.8% | 33.9% | 39.1% | 54.9% | 31 | -2.7% | 6.2% | -14.1% | -7.6% | -2.9% | 0.6% | 10.7% |
| | *month 6* | 36 | 37.3% | 6.6% | 26.9% | 32.3% | 36.3% | 40.3% | 57.1% | 33 | -0.6% | 8.3% | -16.5% | -4.7% | -1.0% | 3.4% | 23.4% |

No statistically significant differences. Only changes from baseline to month 6 were statistically compared. n: number of observations; Mean: the average energy intake or change in energy intake calculated based on the number of participants in the corresponding group and corresponding visit displayed in columns "Product" and "Visit". SD: Standard deviation; Min: Minimum value; Q1: First quartile; Q3: Third quartile; Max: Maximum value.

## Claims

1. A non-therapeutic use of a bacterium of the genus *Bifidobacterium* or a mixture thereof for reducing food, energy and/or fat intake in a mammal, wherein the bacterium of the genus *Bifidobacterium* is the *Bifidobacterium animalis* ssp. *lactis* strain 420 (B420).

2. The use according to any one claim 1, wherein the bacterium is used in combination with one or more fibres and/or prebiotics.

3. The use according to claim 2, wherein the fibres and/or prebiotic is polydextrose.

4. The use according to claims 1-3, wherein the bacterium is in the form of a food product, a dietary supplement or a pharmaceutically acceptable formulation.

## Patentansprüche

1. Nicht therapeutische Verwendung eines Bakteriums der Gattung *Bifidobacterium* oder eines Gemischs davon zur Verringerung der Nahrungs-, Energie- und/oder Fettaufnahme bei einem Säuger, wobei es sich bei dem Bakterium der Gattung *Bifidobacterium* um den Stamm *Bifidobacterium animalis* ssp. *lactis* 420 (B420) handelt.

2. Verwendung nach einem Anspruch 1, wobei das Bakterium in Kombination mit einem oder mehreren Ballaststoffen und/oder Präbiotika verwendet wird.

3. Verwendung nach Anspruch 2, wobei es sich bei den Ballaststoffen und/oder dem Präbiotikum um Polydextrose handelt.

4. Verwendung nach Anspruch 1-3, wobei das Bakterium in Form eines Lebensmittelprodukts, eines Nahrungsergänzungsmittels oder einer pharmazeutisch unbedenklichen Formulierung vorliegt.

## Revendications

1. Utilisation non thérapeutique d'une bactérie du genre *Bifidobacterium* ou d'un mélange correspondant pour la réduction de l'absorption de nourriture, d'énergie et/ou de graisse chez un mammifère, la bactérie du genre *Bifidobacterium* étant la souche *Bifidobacterium animalis* ssp. *lactis* 420 (B420).

2. Utilisation selon une quelconque revendication 1, la bactérie étant utilisée en combinaison avec un(e) ou plusieurs fibres et/ou prébiotiques.

3. Utilisation selon la revendication 2, les fibres et/ou le prébiotique étant un polydextrose.

4. Utilisation selon les revendications 1 à 3, la bactérie étant sous la forme d'un produit alimentaire, d'un supplément diététique ou d'une formulation pharmaceutiquement acceptable.
